# EUROPEAN PATENT APPLICATION

(11) **EP 2 249 149 A1**
(43) Date of publication of application: **10.11.2010**
(21) Application number: 09709129.2
(22) Date of filing: 30.01.2009
(51) Int. Cl.: G01N 25/18, G01N 9/36, G01N 33/22

(54) **FLUID IDENTIFYING METHOD AND FLUID IDENTIFYING APPARATUS**

(30) Priority: 04.02.2008 JP 2008023978
(71) Applicant: Mitsui Mining and Smelting Co., Ltd., Shinagawa-ku Tokyo 141-8584 (JP)
(72) Inventor: YANAGI, Kiyotaka, Ageo-shi Saitama 362-0021 (JP); TSURUDA, Mayuko, Ageo-shi Saitama 362-0021 (JP)
(74) Representative: Gardiner, Stephen Robin
(86) International application number: PCT/JP2009/051634
(87) International publication number: WO 2009/099014

(57) **Abstract**

It is an object to provide a fluid discrimination apparatus and a fluid discrimination method for carrying out with accuracy a fluid discrimination such as a fluid type discrimination, a concentration discrimination, the fluid existence or nonexistence discrimination, and a fluid temperature discrimination for a discriminated fluid that is stored in a tank or the like. A characteristic measured value as an index value that indicates the characteristics of the fluid to be discriminated is measured, and a discrimination of a fluid is carried out by comparing the fluid discrimination data that indicates the relationship between a fluid that has been measured in advance and a characteristic value of the fluid with the characteristic measured value.

## Description

### TECHNICAL FIELD

The present invention relates to a fluid discrimination method and a fluid discrimination apparatus for carrying out a fluid discrimination such as the fluid type discrimination, the concentration discrimination, the fluid existence or nonexistence discrimination, and the fluid temperature discrimination for a fluid to be discriminated.

### BACKGROUND ART

While a fuel to be-used is assumed, an internal combustion engine of an automobile is designed in such a manner that the automobile is optimally operated in the case in which the fuel is used. For instance, a diesel engine is designed in such a manner that an automobile is optimally operated by using the light oil as a fuel. However, an automobile can be operated even in the case in which a fuel other than the light oil, for instance a wide variety of liquid fuels such as a kerosene and a heavy oil, is used.

Consequently, in the case in which a diesel engine of a construction machine or a heavy machine is operated, a liquid fuel which is comparatively moderate in price as compared with a kerosene, a light oil, and a heavy oil is mixed to a light oil to be used in some cases.

However, in the case in which the kerosene having a lubricating property (a viscous property) lower than that of a light oil is mixed to be used, a part of a diesel engine is worn away. Moreover, in the case in which the diesel engine is used over a long period of time, a failure of the diesel engine may occur.

### DISCLOSURE OF THE INVENTION

### PROBLEMS TO BE SOLVED BY THE INVENTION

For a construction machine and a heavy machine in particular, a user uses a construction machine or a heavy machine on lease from a trader of a construction machine and a heavy machine as a practical matter. Consequently, after a construction machine or a heavy machine is returned from a user to a lease trader, a failure of an engine may occur in some cases.

In such a case, since a failure of an engine does not occur during use, it is difficult for a lease trader to pursue user's responsibility for the failure in some cases.

Consequently, a request for a sensor that monitors a type of oil in a fuel tank has been increasing.

### MEANS FOR SOLVING THE PROBLEMS

The present invention was made in order to solve the above problems of the conventional art and to achieve the purpose.
A fluid discrimination method for discriminating a fluid to be discriminated in accordance with the present invention is characterized by comprising the steps of:
measuring a characteristic measured value as an index value that indicates the characteristics of the fluid to be discriminated; and
carrying out a discrimination of a fluid by comparing the fluid discrimination data that indicates the relationship between a fluid that has been measured in advance and a characteristic value of the fluid with the characteristic measured value.

A fluid discrimination apparatus for discriminating a fluid to be discriminated in accordance with the present invention is characterized by comprising:
a characteristic measuring part for measuring a characteristic measured value as an index value that indicates the characteristics of the fluid to be discriminated; and
a discrimination control part that carries out a discrimination of a fluid based on the characteristic measured value,
wherein a discrimination of a fluid is carried out by comparing the fluid discrimination data that is stored into the discrimination control part with the characteristic measured value.

The fluid discrimination method and apparatus in accordance with the present invention are **characterized in that** the index value is at least any one of a specific gravity, a coefficient of kinematic viscosity, and a lubricity (HFRR).

The fluid discrimination apparatus in accordance with the present invention is **characterized in that** the fluid discrimination data is data that indicates the relationship between a fluid that has been measured in advance and an electrical output value that is measured as an index value that indicates the characteristics of the fluid.

The fluid discrimination method in accordance with the present invention is **characterized in that** a voltage is applied to a fluid discrimination element that includes a temperature sensing element for a prescribed time to heat a fluid to be discriminated, and the characteristic measured value is an electrical output value that is output by a fluid discrimination sensor that outputs an electrical output value corresponded to a temperature of the fluid discrimination element.

The fluid discrimination apparatus in accordance with the present invention is **characterized in that**:
the characteristic measuring part is provided with a fluid discrimination element that includes a temperature sensing element and a fluid discrimination sensor that outputs an electrical output value corresponded to a temperature of the fluid discrimination element in which a voltage is applied to the fluid discrimination element for a prescribed time to heat a fluid to be discriminated,
the discrimination control part carries out a discrimination of a fluid based on the electrical output value, and
the discrimination control part carries out a discrimination of a fluid by comparing the electrical output value with the fluid discrimination data that is stored into the discrimination control part in advance.

The fluid discrimination method in accordance with the present invention is **characterized in that** a voltage is applied to a fluid discrimination element that includes a temperature sensing element to heat a fluid to be discriminated, and the characteristic measured value is an electrical output value that is based on the voltage and that is output by a fluid discrimination sensor that controls the voltage in such a manner that a temperature of the fluid discrimination element is a constant value.

The fluid discrimination apparatus in accordance with the present invention is **characterized in that**:
the characteristic measuring part is provided with a fluid discrimination element that includes a temperature sensing element and a fluid discrimination sensor that controls the voltage in such a manner that a temperature of the fluid discrimination element is a constant value and that outputs an electrical output value that is based on the voltage in which a voltage is applied to the fluid discrimination element to heat a fluid to be discriminated,
the discrimination control part carries out a discrimination of a fluid.based on the electrical output value, and
the discrimination control part carries out a discrimination of a fluid by comparing the electrical output value with the fluid discrimination data that is stored into the discrimination control part in advance.

The fluid discrimination method and apparatus in accordance with the present invention are **characterized in that** the fluid discrimination element is provided with an electrical heating element and a temperature sensing element that is disposed close to the electrical heating element.

The fluid discrimination method and apparatus in accordance with the present invention are **characterized in that** the temperature sensing element of the fluid discrimination element is a temperature sensing element that is provided with a heating function and a temperature sensing function.

The fluid discrimination method and apparatus in accordance with the present invention are **characterized in that** the fluid discrimination sensor is provided with the fluid discrimination element and a fluid temperature detecting element that is disposed separately at a regular interval from the fluid discrimination element.

The fluid discrimination method and apparatus in accordance with the present invention are **characterized in that** the fluid discrimination element and the fluid temperature detecting element are disposed horizontally to a fluid level.

The fluid discrimination method and apparatus in accordance with the present invention are **characterized in that** the discrimination of a fluid to be discriminated is at least one of a fluid type discrimination, a concentration discrimination, the fluid existence or nonexistence discrimination, and a fluid temperature discrimination.

The fluid discrimination method and apparatus in accordance with the present invention are **characterized in that** the fluid to be discriminated is a hydrocarbon series fluid.

The fluid discrimination method and apparatus in accordance with the present invention are **characterized in that** the fluid to be discriminated is at least one of a light oil, a kerosene, and a heavy oil.

The fluid discrimination method and apparatus in accordance with the present invention are **characterized in that** the fluid to be discriminated is at least one of a light oil, a kerosene, and a heavy oil that are contained in a lubricating oil that is stored into an oil tank.

### EFFECT OF THE INVENTION

By the present invention, a characteristic measured value that indicates the characteristics of the fluid to be discriminated is measured, and a discrimination of a fluid can be carried out by comparing the fluid discrimination data that indicates the relationship between a fluid that has been measured in advance and a characteristic value of the fluid with the characteristic measured value. Consequently, it is possible to carry out with accuracy a fluid discrimination such as a fluid type discrimination, a concentration discrimination, the fluid existence or nonexistence discrimination, and a fluid temperature discrimination for a fluid to be discriminated.

### BRIEF DESCRIPTION OF THE DRAWINGS

Fig. 1 is an exploded view showing a first embodiment of a fluid discrimination apparatus in accordance with the present invention.
Fig. 2 is an enlarged view showing a fluid discrimination sensor module of the fluid discrimination apparatus of Fig. 1.
Fig. 3 is a schematic cross sectional view showing the fluid discrimination apparatus of Fig. 1.
Fig. 4 is a schematic cross sectional view showing a usage state of the fluid discrimination apparatus in accordance with the first embodiment.
Fig. 5 is a schematic view showing a usage state of the fluid discrimination apparatus in accordance with the first embodiment.
Fig. 6 is an exploded schematic view showing a thin film chip of a fluid discrimination element 21.
Fig. 7 is a circuit block diagram for a fluid discrimination.
Fig. 8 is a view showing a relationship between a single pulse voltage P that is applied to an electrical heating element and a sensor output Q.
Fig. 9 is a statistical chart showing a relationship between an average sensor output voltage value V1 that has been obtained by using the fluid discrimination apparatus in accordance with the embodiment of the present invention and a specific gravity of a fluid to be discriminated.
Fig. 10 is a statistical chart showing a relationship between an average sensor output voltage value V1 that has been obtained by using the fluid discrimination apparatus in accordance with the embodiment of the present invention and lubricity (HFRR) of a fluid to be discriminated.
Fig. 11 is a statistical chart showing a relationship between an average sensor output voltage value V1 that has been obtained by using the fluid discrimination apparatus in accordance with the embodiment of the present invention and a coefficient of kinematic viscosity of a fluid to be discriminated.
Fig. 12 is a statistical chart in which a relationship between an average sensor output voltage value V1 and a coefficient of kinematic viscosity of a fluid to be discriminated for water as a fluid to be discriminated has been added to the graph of Fig. 11.
Fig. 13 is a statistical chart in which a relationship between an average sensor output voltage value V1 and a specific gravity of a fluid to be discriminated for an air as a fluid to be discriminated has been added to the graph of Fig. 9.
Fig. 14 is a circuit block diagram showing an ASIC 52 for a second embodiment of a fluid discrimination apparatus 10 in accordance with the present invention.
Fig. 15 is a view showing a relationship between an applied voltage P that is applied to an electrical heating element and a sensor output Q.
Fig. 16 is a statistical chart showing a relationship between an average sensor output voltage value V2 that has been obtained by using the fluid discrimination apparatus in accordance with the embodiment of the present invention and a specific gravity of a fluid to be discriminated.
Fig. 17 is a statistical chart showing a relationship between an average sensor output voltage value V2 that has been obtained by using the fluid discrimination apparatus in accordance with the embodiment of the present invention and lubricity (HFRR) of a fluid to be discriminated.
Fig. 18 is a statistical chart showing a relationship between an average sensor output voltage value V2 that has been obtained by using the fluid discrimination apparatus in accordance with the embodiment of the present invention and a coefficient of kinematic viscosity of a fluid to be discriminated.
Fig. 19 is a statistical chart in which a relationship between an average sensor output voltage value V1 and a coefficient of kinematic viscosity of a fluid to be discriminated for water as a fluid to be discriminated has been added to the graph of Fig. 18.
Fig. 20 is a schematic view showing a third embodiment of a fluid discrimination apparatus in accordance with the present invention.
Fig. 21 is a schematic view showing an example of a usage state of the fluid discrimination apparatus of Fig. 20.
Fig. 22 is a schematic view showing an example of another usage state of the fluid discrimination apparatus of Fig. 20.
Fig. 23 is a circuit block diagram showing a configuration of another circuit of an ASIC 52 for a fluid discrimination as a fourth embodiment of a fluid discrimination apparatus in accordance with the present invention.

### EXPLANATIONS OF LETTERS OR NUMERALS

10: Fluid discrimination apparatus
11: Fluid discrimination apparatus
12: support part
16: Water proof case
20: Fluid discrimination sensor module
21: Fluid discrimination element
21a: Fluid detecting thin film chip
21a1: Chip substrate
21a2: Fluid detecting temperature sensing element
21a2: Temperature sensing element
21a3: Interlayer insulation film
21a4: Electrical heating element
21a5: Electrical heating element electrode
21a6: Protective film
21a7: Electrode pad
21e: External electrode terminal
22: Fluid temperature detecting element
22a2: Temperature sensing element
22e: External electrode terminal
26: Mold resin
36: Cover member
38: Discriminated fluid introduction path
40: Power cable
42: Communication cable
49: Communication power connector
50: Control unit
52: ASIC
54: Storage device
56: Power connection terminal
58: CAN interface
64: Resistor
65: Resistor
68: Bridge circuit
70: Differential amplifier
71: Fluid temperature detecting amplifier
72: Microcomputer
74: Switch
76: Output buffer circuit
100: Fuel tank

### BEST MODE OF CARRYING OUT THE INVENTION

An embodiment (example) of the present invention will be described below in detail with reference to the drawings.

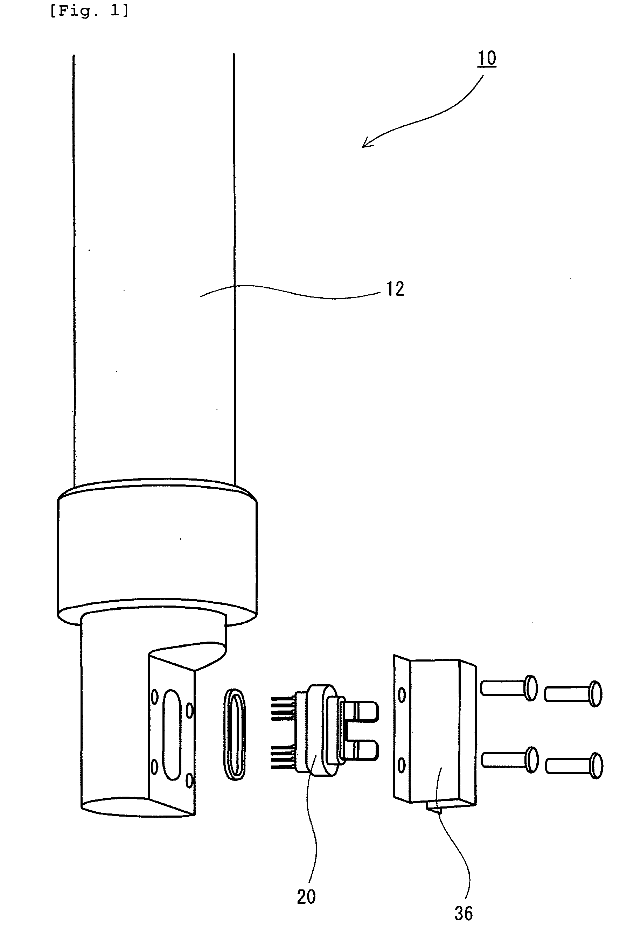

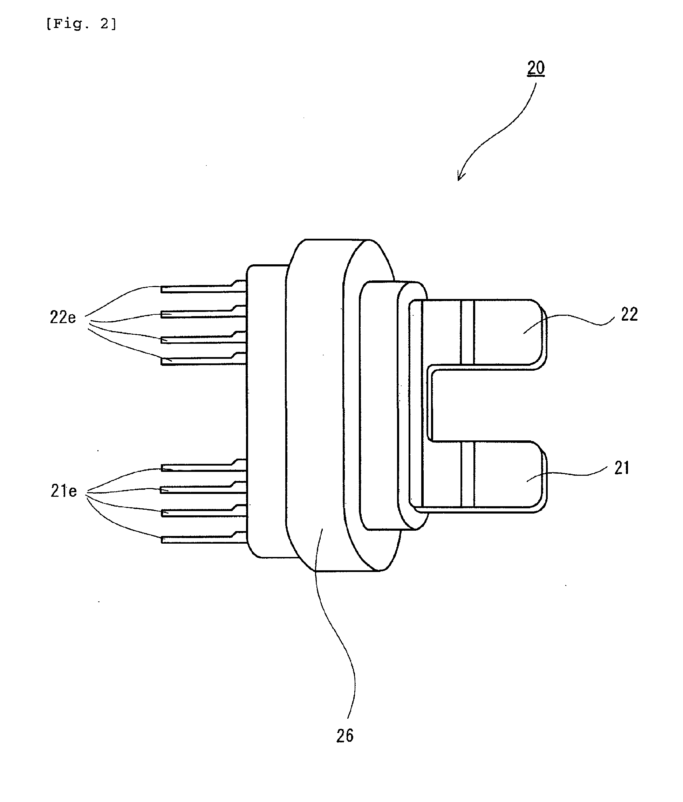

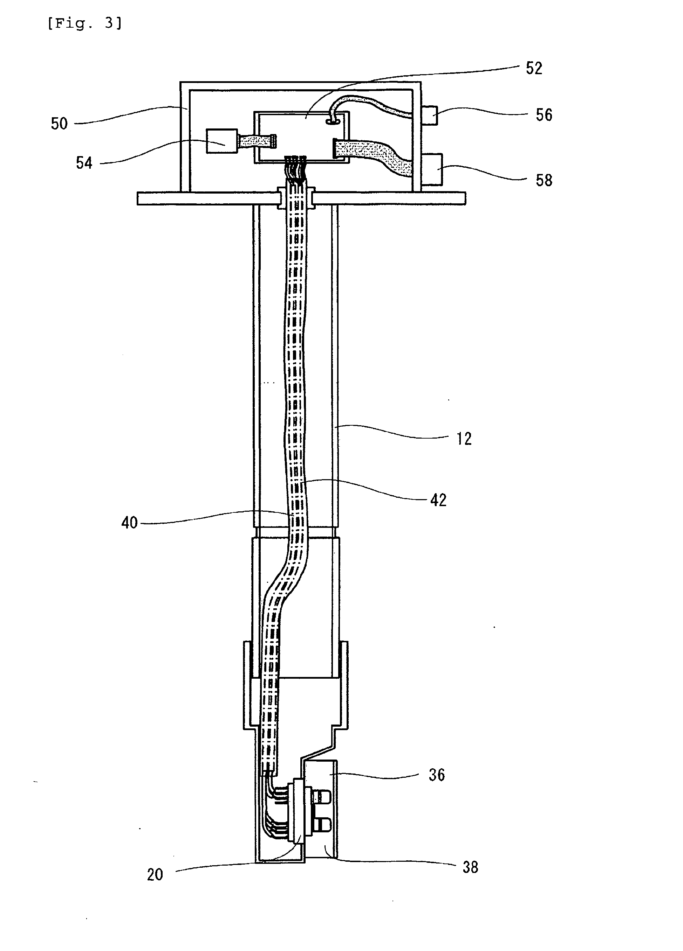

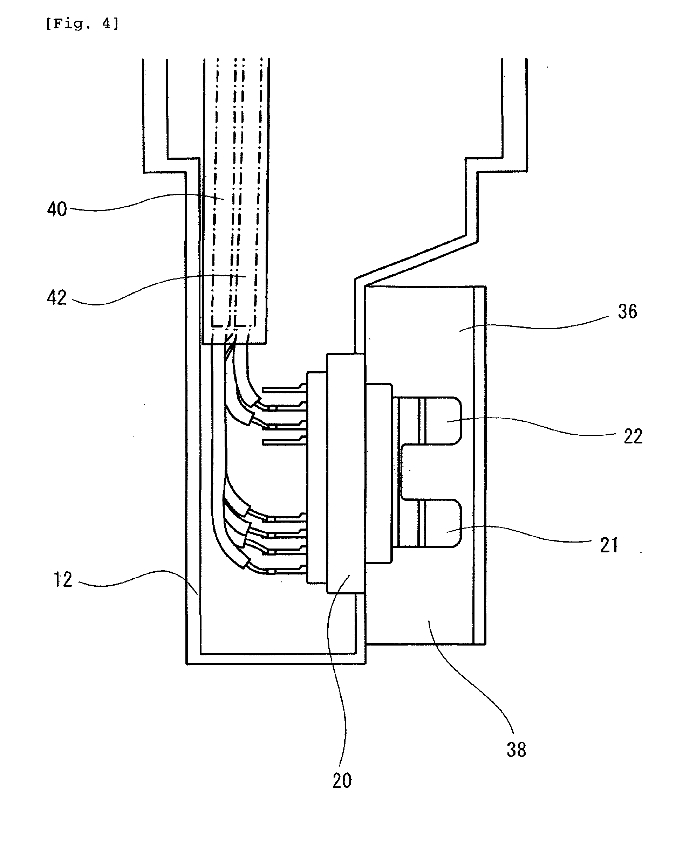

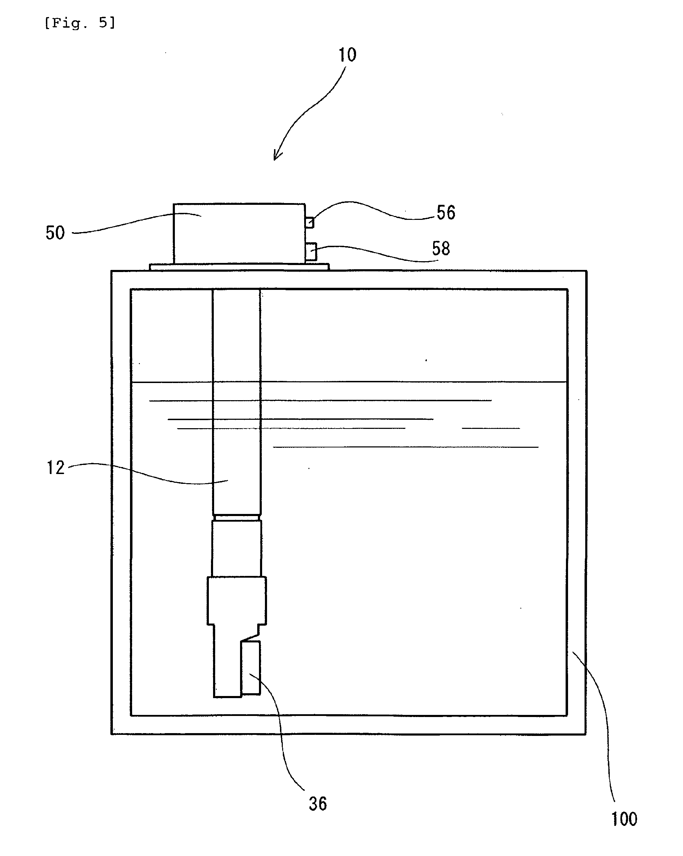

Fig. 1 is an exploded view showing a first embodiment of a fluid discrimination apparatus in accordance with the present invention. Fig. 2 is an enlarged view showing a fluid discrimination sensor module of the fluid discrimination apparatus of Fig. 1. Fig. 3 is a schematic cross sectional view showing the fluid discrimination apparatus of Fig. 1. Fig. 4 is a schematic cross sectional view showing a usage state of the fluid discrimination apparatus in accordance with the present embodiment. Fig. 5 is a schematic view showing a usage state of the fluid discrimination apparatus in accordance with the present embodiment.

As shown in Fig. 3, a fluid discrimination apparatus 10 in accordance with an embodiment of the present invention is provided with a support part 12 and is attached to a fuel tank 100 that is mounted to an automobile and a construction machine such as a transporting machine, a bulldozer, and a crane for instance.

Moreover, as shown in Figs. 1 and 4, the fluid discrimination apparatus 10 in accordance with an embodiment of the present invention is provided with a fluid discrimination sensor module 20, a cover member 36, a power cable 40, and a communication cable 42. The fluid discrimination sensor module 20 is disposed in such a manner that only a side on which a fluid discrimination element 21 and a fluid temperature detecting element 22 are disposed (hereafter referred to a fluid exposed side) comes into contact with a fluid.

As shown in Fig. 2, the fluid discrimination sensor module 20 is molded by a mold resin 26 in an integrated manner in a state in which the fluid discrimination element 21 and the fluid temperature detecting element 22 are disposed separately at a regular interval from each other. A numeral 21e represents an external electrode terminal that is electrically connected to the fluid discrimination element 21, and a numeral 22e represents an external electrode terminal that is electrically connected to the fluid temperature detecting element 22.

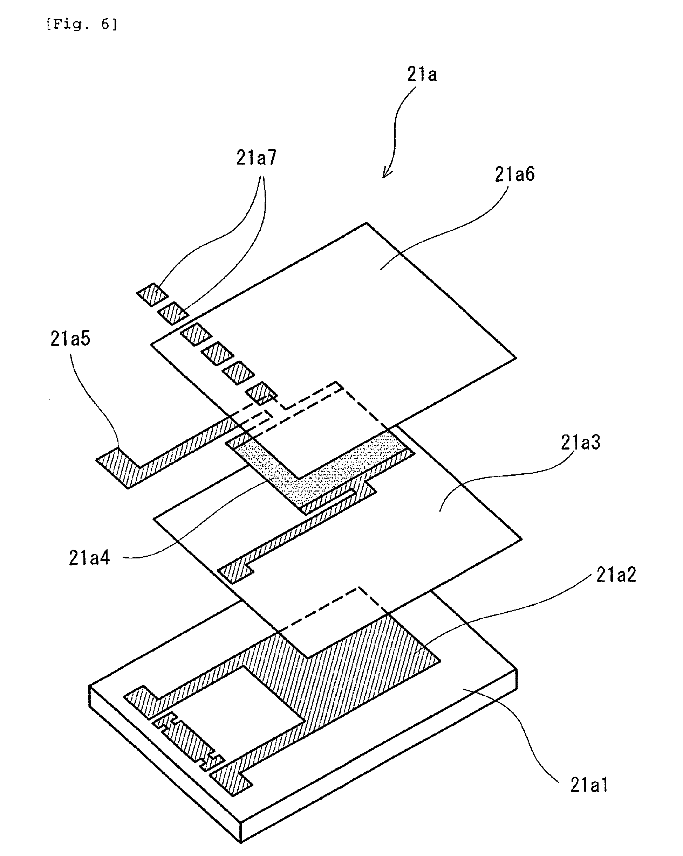

In the embodiment of the present invention, as shown in Fig. 6, the fluid discrimination element 21 is configured by a fluid detecting thin film chip 21a in which a fluid detecting temperature sensing element is formed by a thin film on a chip substrate.

The fluid detecting thin film chip 21a is composed of a chip substrate 21a1 made of Al₂O₃, a fluid detecting temperature sensing element 21a2 made of Pt, an interlayer insulation film 21a3 made of SiO₂, an electrical heating element 21a4 made of TaSiO₂ an electrical heating element electrode 21a5 made of Ni, a protective film 21a6 made of SiO₂, and an electrode pad 21a7 made of Ti/Au, which are laminated in the order as needed for instance. Although this is not shown in the figure, the fluid detecting temperature sensing element 21a2 is formed in a meandering pattern.

The electrode pad 21a7 that is connected to the fluid detecting temperature sensing element 21a2 and the electrical heating element electrode 21a5 is connected to the external electrode terminal 21e via a bonding wire (not shown).

Moreover, the fluid temperature detecting element 22 can also be configured similarly to the fluid discrimination element 21. However, only a temperature sensing element (a fluid temperature detecting temperature sensing element for the fluid temperature detecting element 22) is operated without operating an electrical heating element. A fluid temperature detecting element 22 in which an electrical heating element and an electrical heating element electrode are not formed can also be used unlike a fluid temperature detecting element for a fluid discrimination element.

Moreover, as shown in Figs. 3 and 4, the external electrode terminal 21e of the fluid discrimination element 21 and the external electrode terminal 22e of the fluid temperature detecting element 22 are connected to a power cable 40 and a communication cable 42, respectively.

The power cable 40 and the communication cable 42 are extended upward through the inside of the support part 12, and are connected to a control unit 50 that is disposed outside the fuel tank 100 and that configures a discrimination control part.

The control unit 50 is provided with an ASIC (Application Specific Integrated Circuit) 52 that carries out a control of voltage application to the fluid discrimination sensor module 20 and a discrimination of a fluid based on an electrical output of the fluid discrimination sensor module 20, a storage device 54 for stores the fluid discrimination data that has been measured in advance, a power connection terminal 56 for a power input, and a CAN interface 58 for carrying out a CAN (Cable Area Network) communication.

Moreover, as shown in Figs. 1 and 4, a cover member 36 is attached to the fluid discrimination apparatus 10 in such a manner that the fluid discrimination sensor module 20 is surrounded by the cover member 36. A discriminated fluid introduction path 38 that has the both upper and lower ends opened and that is extended in a vertical direction through a region close to a fluid exposed side of the fluid discrimination sensor module 20 is formed by the cover member 36.

In the embodiment of the present invention, the fluid discrimination element 21 and the fluid temperature detecting element 22 of the fluid discrimination sensor module 20 are disposed vertically to a fluid level, and the discriminated fluid introduction path 38 has the both upper and lower ends that are opened. However, the fluid discrimination element 21 and the fluid temperature detecting element 22 of the fluid discrimination sensor module 20 can also be disposed horizontally to a fluid level, and the discriminated fluid introduction path 38 can also have the both right and left ends that are opened.

By horizontally disposing the fluid discrimination element 21 and the fluid temperature detecting element 22 as described above, a difference between a temperature distribution of a fluid to be discriminated around the fluid discrimination element 21 and a temperature distribution of a fluid to be discriminated around the fluid temperature detecting element 22 can be reduced.

The fluid discrimination apparatus in accordance with the present invention carries out a discrimination of a fluid based on a temperature change of a fluid to be discriminated as described later. Consequently, by disposing the fluid discrimination element 21 and the fluid temperature detecting element 22 horizontally to a fluid level, a difference in a temperature distribution can be reduced, thereby improving the discrimination accuracy.

The fluid discrimination apparatus 10 that is configured as described is used in the state in which the fluid discrimination apparatus 10 is attached to a fuel tank 100 as shown in Fig. 5 for instance.

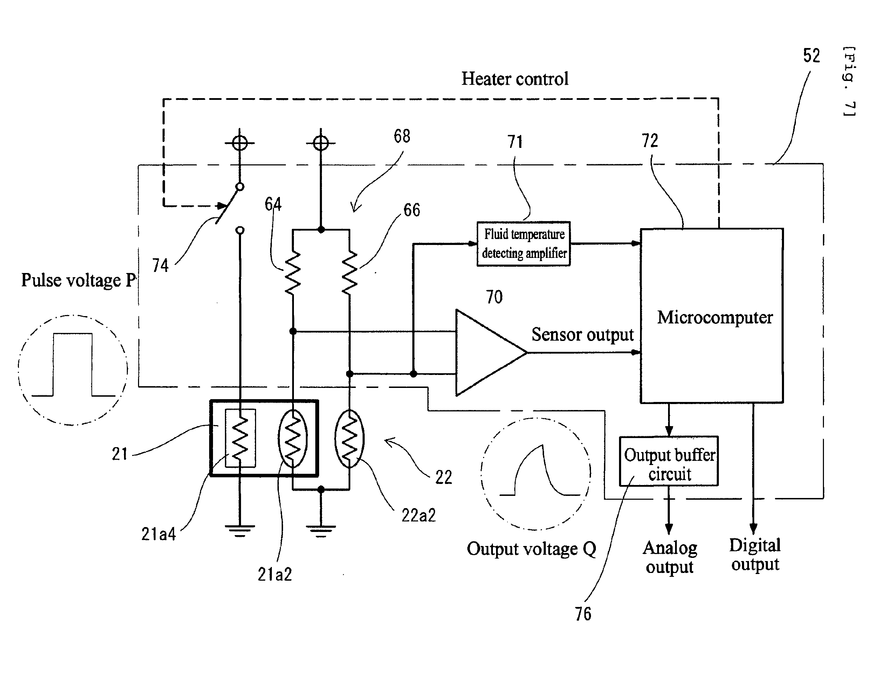

Fig. 7 is a circuit block diagram for showing a circuit configuration of an ASIC 52 for a fluid discrimination in accordance with the embodiment of the present invention. A bridge circuit (a fluid discrimination circuit) 68 is configured by the temperature sensing element 21a2 of the fluid discrimination element 21, the temperature sensing element 22a2 of the fluid temperature detecting element 22, and two resistors 64 and 66. An output of the bridge circuit 68 is input to a differential amplifier 70, and an output of the differential amplifier 70 (also called a fluid discrimination circuit output or a sensor output) is input to a microcomputer 72 that configures a computing part via an A/D converter.

A fluid temperature corresponding output value that is corresponded to a temperature of a fluid to be discriminated is input from the temperature sensing element 22a2 of the fluid temperature detecting element 22 to the microcomputer 72 via a fluid temperature detecting amplifier 71. On the other hand, the microcomputer 72 outputs a heater control signal that controls the opening and closing of a switch 74 to the switch 74 that is located a power distribution path to the electrical heating element 21a4 of the fluid discrimination element 21.

After a fluid discrimination is carried out by the microcomputer 72 as described later, a discrimination signal that indicates a fluid type, a concentration, the existence or nonexistence of a fluid, and a temperature of a fluid is output as an analog signal via a D/A converter (not shown) and a buffer circuit 76. The discrimination signal can also be output as a digital signal. An analog output and a digital output can be selected depending on an apparatus that receives the discrimination signal.

A part that is surrounded by an alternate long and short dash line in Fig. 7 is formed in the ASIC 52.

Fig. 7 shows a configuration in which the switch 74 is simply opened and closed as a matter of practical convenience. However, a plurality of voltage application paths that can apply voltages different from each other can also be formed in a fabrication of the ASIC 52, and any of the voltage application paths can be selected in the case in which a heater is controlled.

By the above configuration, a range of selecting a characteristic of the electrical heating element 21a4 of the fluid discrimination element 21 can be extremely enlarged. In other words, a voltage that is optimum for a measurement can be applied corresponding to the characteristics of the electrical heating element 21a4. Moreover, a plurality of voltage applications different from each other can be carried out in the case in which a heater is controlled, whereby a range of types of a fluid to be discriminated can be enlarged.

Fig. 7 shows the resistors 64 and 66 having a fixed resistance value as a matter of practical convenience. However, variable resistors can be formed as the resistors 64 and 66 in the case in which the ASIC 52 is formed, and the resistance values of the resistors 64 and 66 can be changed as needed in a measurement.

Similarly, the differential amplifier 70 and the fluid temperature detecting amplifier 71 can be formed in such a manner that the characteristics of the differential amplifier 70 and the fluid temperature detecting amplifier 71 can be adjusted in the case in which the ASIC 52 is formed, and the characteristics of the amplifiers can be changed as needed in a measurement.

By the above configuration, the characteristics of the fluid discrimination circuit can be easily set to be optimum, and a dispersion of the measurement characteristics, which occurs based on an individual dispersion on a production of the fluid discrimination element 21 and the fluid temperature detecting element 22 and an individual dispersion on a production of the ASIC 52, can be reduced, thereby improving a production yield.

As an example of the fluid discrimination apparatus in accordance with the embodiment of the present invention, an oil type discrimination operation of a light oil, a kerosene, and a heavy oil will be described in the following.

In the case in which a fuel F to be discriminated is stored into the fuel tank 100, the fuel F to be discriminated is also filled with in the discriminated fluid introduction path 38 that is formed by the cover member 36 that covers the fluid discrimination sensor module 20. The fuel F to be discriminated that has been stored into the fuel tank 100 and the discriminated fluid introduction path 38 does not flow in substance.

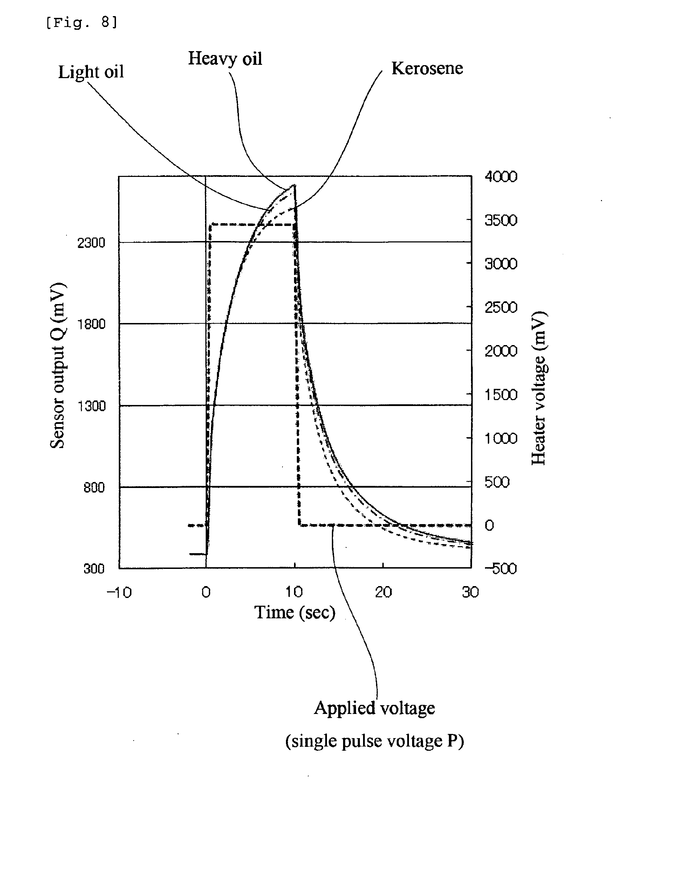

The switch 74 is closed for a prescribed time (10 seconds for instance) by a heater control signal that is output from the microcomputer 72 to the switch 74, and a single pulse voltage P of a prescribed height (3.45 V for instance) is applied to the electrical heating element 21a4 to make the electrical heating element generate a heat. As shown in Fig. 8, an output voltage (a sensor output) of the differential amplifier 70 at this time is increased by a gradual process in a voltage application to the electrical heating element 21a4, and is decreased by a gradual process after a voltage application to the electrical heating element 21a4 is completed.

As shown in Fig. 8, immediately before a voltage application to the electrical heating element 21a4 is completed, the microcomputer 72 carries out a sampling of prescribed numbers (256 times for instance) and carries out an operation for getting the average value to obtain an average sensor output voltage value V1. The average sensor output voltage value V1 is corresponded to a peak temperature of the temperature sensing element 21a2.

It is not always necessary that an average sensor output voltage value V1 is a value that is corresponded to a peak temperature of the temperature sensing element 21a2. The microcomputer 72 can also carry out a sampling of prescribed numbers for a sensor output after a prescribed time (5 seconds for instance) elapses from a start of a voltage application to the electrical heating element 21a4, and can carry out an operation for getting the average value to obtain an average sensor output voltage value.

For the meanwhile, a heat that has been generated by the electrical heating element 21a4 based on a voltage application of a single pulse as described above is transferred to a fluid to be discriminated. By this heat transfer, a fluid to be discriminated around the fluid discrimination sensor module 20 is heated, and a temperature of the fluid to be discriminated is increased. The heat transfer depends on a characteristic value of a fluid to be discriminated. A degree of a temperature increase of the temperature sensing element 21a2 is changed by a type and a concentration of a fluid and a temperature of a fluid.

As the characteristic value of a fluid to be discriminated, a specific gravity, a coefficient of kinematic viscosity, and a lubricity (HFRR: High Frequency Reciprocating Rig) of the fluid to be discriminated can be mentioned for instance.

In the case in which the average sensor output voltage value V1 that has been obtained by a measurement using a fluid discrimination apparatus 10 in accordance with the present embodiment and a specific gravity, a coefficient of kinematic viscosity, and a lubricity (HFRR: High Frequency Reciprocating Rig) as the characteristic values of a fluid to be discriminated are used for kerosene, a light oil, a heavy oil, and a mixed oil thereof as a fluid to be discriminated, the fluid discrimination data will be described in the following.

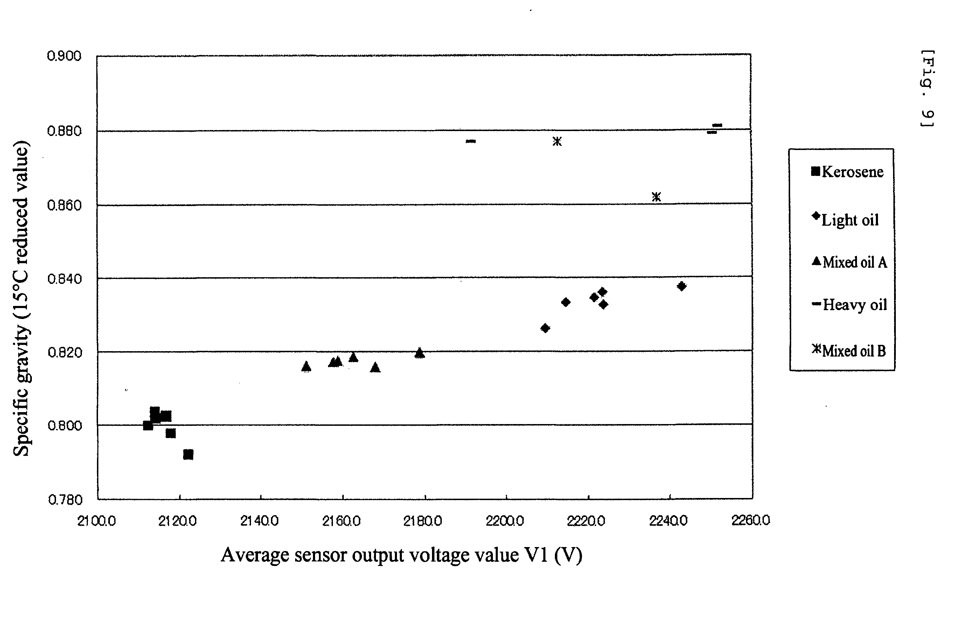

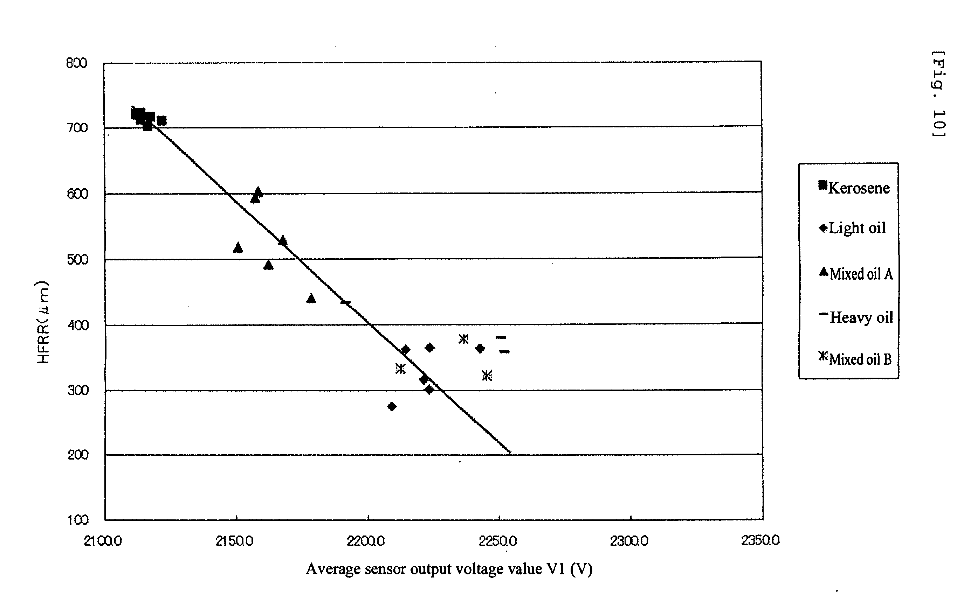

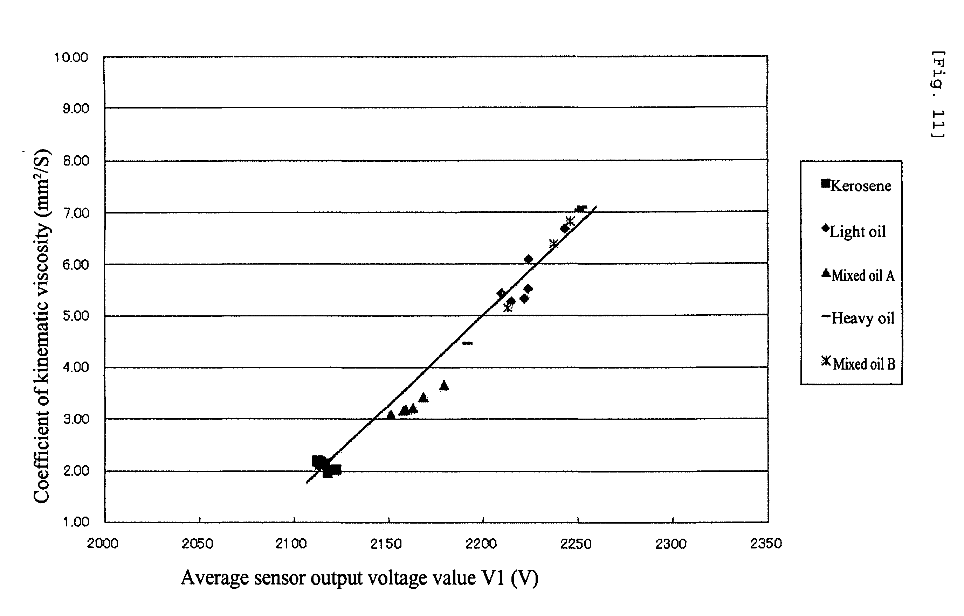

Fig. 9 is a statistical chart showing a relationship between an average sensor output voltage value V1 that has been obtained by using the fluid discrimination apparatus in accordance with the embodiment of the present invention and a specific gravity of a fluid to be discriminated. Fig. 10 is a statistical chart showing a relationship between an average sensor output voltage value V1 that has been obtained by using the fluid discrimination apparatus in accordance with the embodiment of the present invention and lubricity (HFRR) of a fluid to be discriminated. Fig. 11 is a statistical chart showing a relationship between an average sensor output voltage value V1 that has been obtained by using the fluid discrimination apparatus in accordance with the embodiment of the present invention and a coefficient of kinematic viscosity of a fluid to be discriminated.

Fig. 9 is a statistical chart showing a relationship between an average sensor output voltage value V1 and a specific gravity of a fluid to be discriminated for kerosene, a light oil, a mixed oil A in which kerosene and a light oil are mixed at a ratio of 1:1, a heavy oil, and a mixed oil B in which a heavy oil and a light oil are mixed at a ratio of 1:1 as a fluid to be discriminated. As shown by the statistical chart, kerosene, a light oil, and a mixed oil A have a correlation.

Fig. 10 is a statistical chart showing a relationship between an average sensor output voltage value V1 and lubricity (HFRR) of a fluid to be discriminated for kerosene, a light oil, a mixed oil A in which kerosene and a light oil are mixed at a ratio of 1:1, a heavy oil, and a mixed oil B in which a heavy oil and a light oil are mixed at a ratio of 1:1 as a fluid to be discriminated. As shown by the statistical chart, all types of oils that have measured have a correlation.

Fig. 10 is a statistical chart showing a relationship between an average sensor output voltage value V1 and a coefficient of kinematic viscosity of a fluid to be discriminated for kerosene, a light oil, a mixed oil A in which kerosene and a light oil are mixed at a ratio of 1:1, a heavy oil, and a mixed oil B in which a heavy oil and a light oil are mixed at a ratio of 1:1 as a fluid to be discriminated. As shown by the statistical chart, all types of oils that have measured have a correlation.

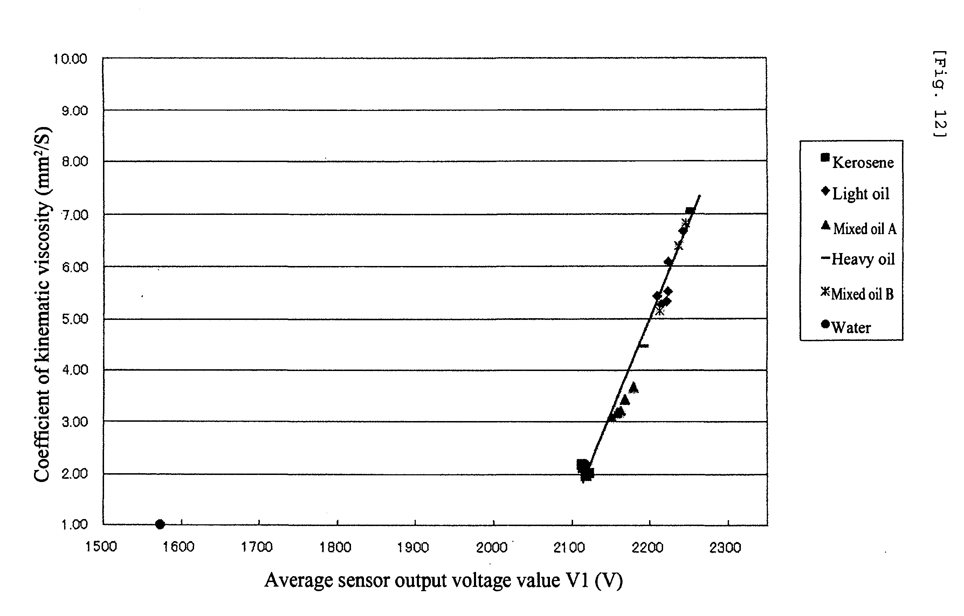

Fig. 12 is a statistical chart in which a relationship between an average sensor output voltage value V1 and a coefficient of kinematic viscosity of a fluid to be discriminated for water as a fluid to be discriminated has been added to the graph of Fig. 11. As shown by the statistical chart, an output that is apparently different from that of an oil such as kerosene and a light oil can be obtained in the case in which a fluid to be discriminated is water, and an output that is apparently smaller than that of an oil can be obtained in the case in which a coefficient of kinematic viscosity is an index value.

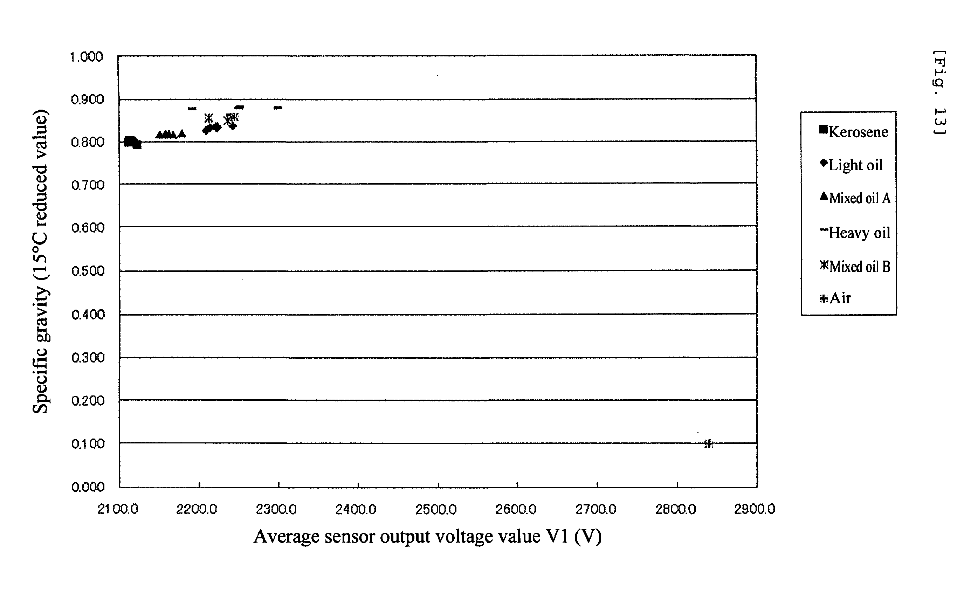

Fig. 13 is a statistical chart in which a relationship between an average sensor output voltage value V1 and a specific gravity of a fluid to be discriminated for an air as a fluid to be discriminated has been added to the graph of Fig. 11. As shown by the statistical chart, an output that is apparently different from that of an oil such as kerosene and a light oil can be obtained in the case in which a fluid to be discriminated is an air, that is, a fuel tank 100 is filled with an air, and an output that is apparently larger than that of an oil can be obtained in the case in which a specific gravity is an index value.

As described above, in the case in which a fluid to be discriminated in the fuel tank 100 is water or the fuel tank 100 is filled with an air, an output as an average sensor output voltage value V1 in the case of an oil is in the constant range, thereby enabling a discrimination of a fluid by setting a threshold value.

The embodiment of the present invention shows the cases in which a specific gravity and a coefficient of kinematic viscosity are used as an index value. However, even for other index values similarly, an output as an average sensor output voltage value V1 in the case of an oil is in the constant range, thereby enabling a discrimination of a fluid by setting a threshold value.

The fluid discrimination data that indicates the relationship between an average sensor output voltage value V1 and a characteristic value of a fluid to be discriminated is stored into a storage device 54 in advance. In the case in which a fluid to be discriminated is then discriminated, a type of a fluid to be discriminated can be discriminated by comparing the fluid discrimination data and the obtained average sensor output voltage value V1 with each other.

In the case in which a fluid to be discriminated is discriminated, the fluid discrimination data of a single index value and the average sensor output voltage value V1 can be compared with each other to discriminate a fluid to be discriminated. However, a discrimination accuracy of a fluid to be discriminated can be improved by comparing the fluid discrimination data of a plurality of index values and the average sensor output voltage value V1 with each other.

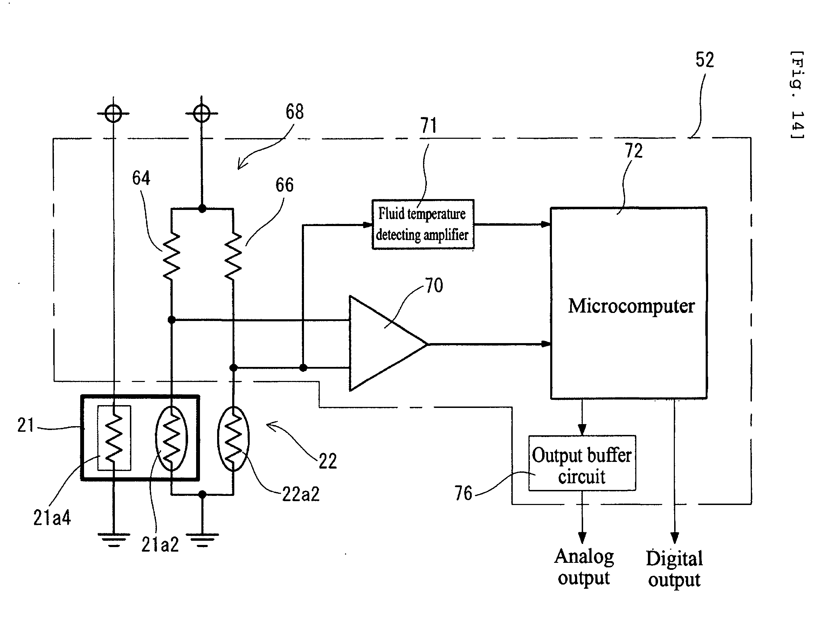

Fig. 14 is a circuit block diagram showing an ASIC 52 for a second embodiment of a fluid discrimination apparatus 10 in accordance with the present invention.

The configuration of a fluid discrimination apparatus 10 in accordance with the present embodiment of the present invention is basically equivalent to the configuration of a fluid discrimination apparatus 10 in accordance with the first embodiment of the present invention, configuration elements equivalent to those of the fluid discrimination apparatus 10 in accordance with the first embodiment of the present invention are numerically numbered similarly and the detailed descriptions of the equivalent elements are omitted.

As shown in Fig. 14, for the ASIC 52 of the second embodiment in accordance with the present invention, since a power distribution control for an electrical heating element 21a4 is not carried out, the ASIC 52 is not provided with a switch 74.

For the present embodiment of the present invention, a temperature of a fluid is measured by the fluid temperature detecting element 22 on a steady basis, a power distribution to the electrical heating element 21a4 is carried out on a steady basis, and an increase in a temperature due to the power distribution can be measured by the fluid discrimination element 21.

An applied voltage is controlled by a control unit 50 in such a manner that a temperature of a fluid is a prescribed value (for instance, a value 10°C higher than a temperature of a fluid before an application of a voltage is started).

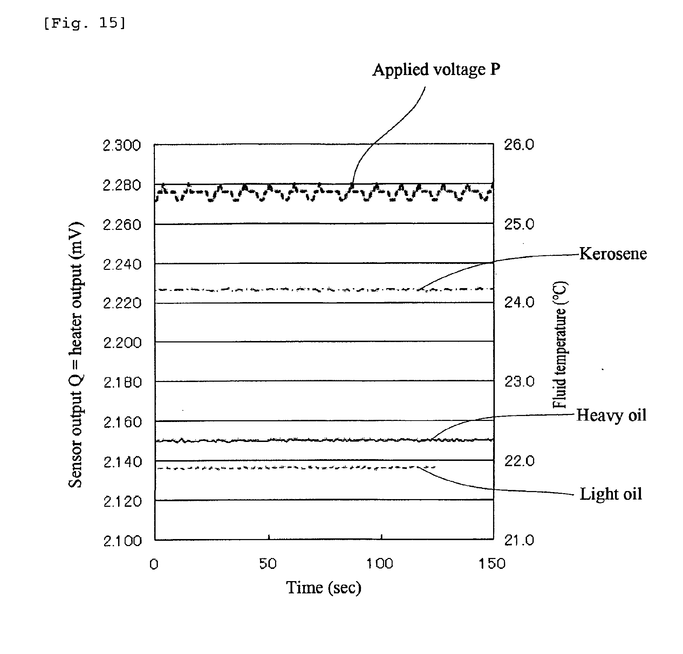

A sensor output Q at this time is output as an almost constant value as shown in Fig. 15. Fig. 15 is a view showing an example of a sensor output Q in the case in which a light oil, a kerosene, and a heavy oil are used as a fluid to be discriminated.

Similarly to the first embodiment, the microcomputer 72 carries out a sampling of prescribed numbers (256 times for instance) for a sensor output Q and carries out an operation for getting the average value to obtain an average sensor output voltage value V2. The average sensor output voltage value V2 is corresponded to a calorific value of the electrical heating element 21a4.

In the case in which the average sensor output voltage value V2 that has been obtained by a measurement using a fluid discrimination apparatus 10 in accordance with the present embodiment and a specific gravity, a coefficient of kinematic viscosity, and a lubricity (HFRR: High Frequency Reciprocating Rig) as the characteristic values of a fluid to be discriminated are used for kerosene, a light oil, a heavy oil, and a mixed oil thereof as a fluid to be discriminated, the fluid discrimination data will be described in the following.

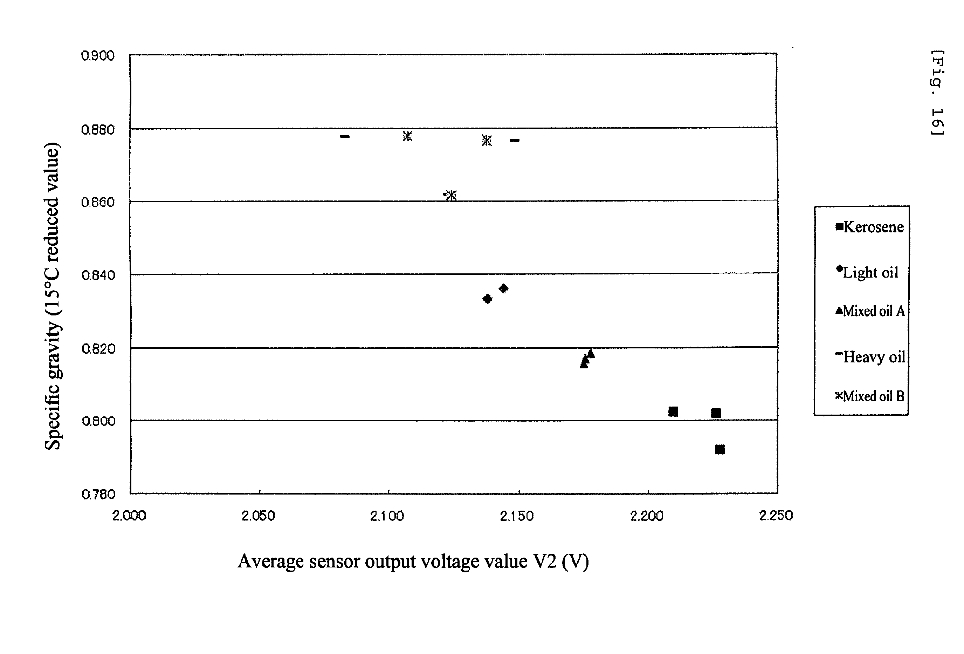

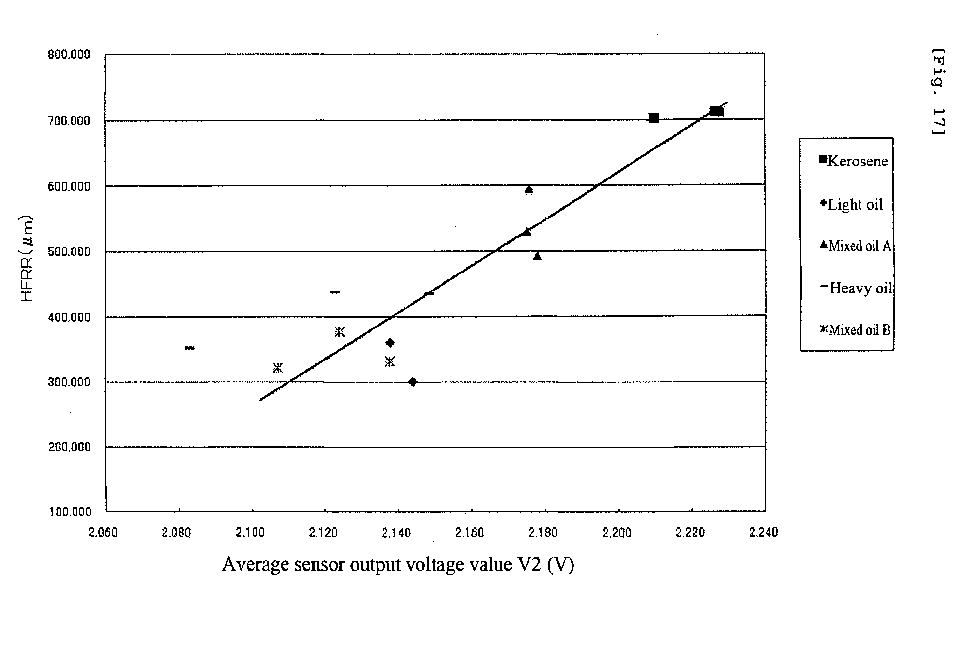

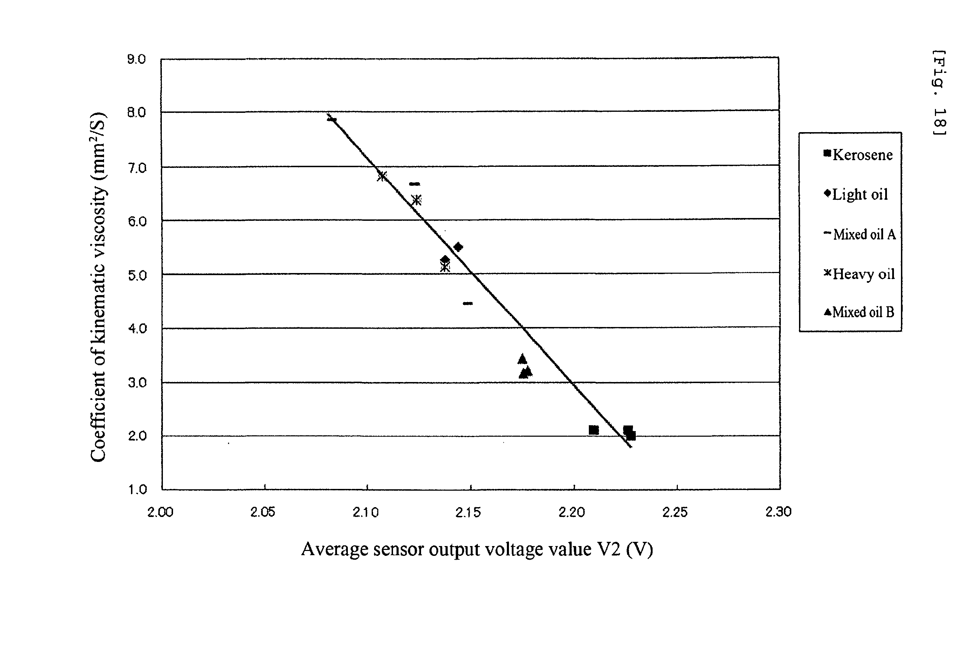

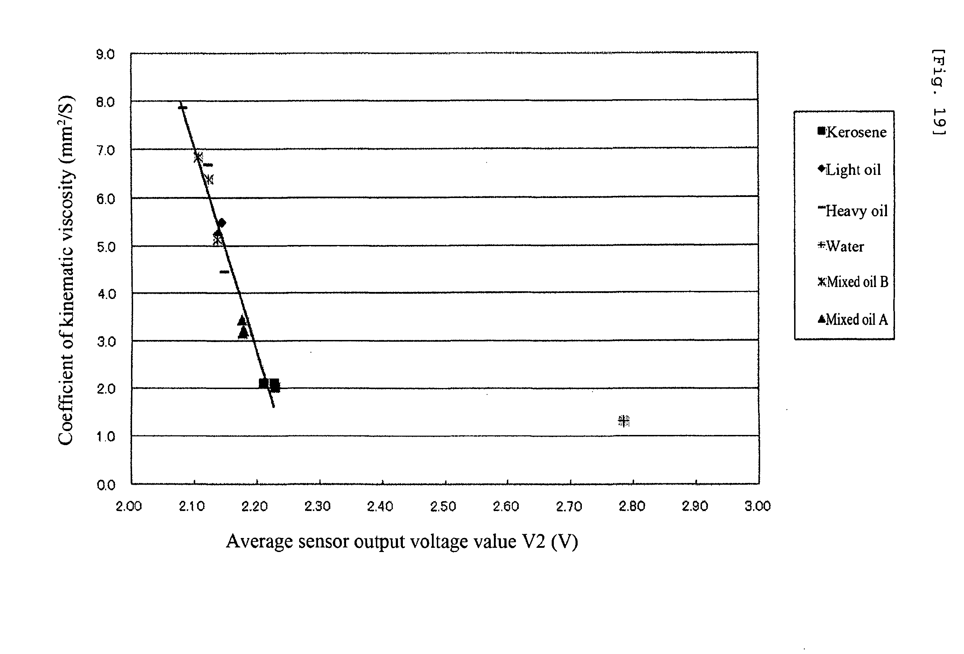

Fig. 16 is a statistical chart showing a relationship between an average sensor output voltage value V2 that has been obtained by using the fluid discrimination apparatus in accordance with the embodiment of the present invention and a specific gravity of a fluid to be discriminated. Fig. 17 is a statistical chart showing a relationship between an average sensor output voltage value V2 that has been obtained by using the fluid discrimination apparatus in accordance with the embodiment of the present invention and lubricity (HFRR) of a fluid to be discriminated. Fig. 18 is a statistical chart showing a relationship between an average sensor output voltage value V2 that has been obtained by using the fluid discrimination apparatus in accordance with the embodiment of the present invention and a coefficient of kinematic viscosity of a fluid to be discriminated. Fig. 19 is a statistical chart in which a part of the statistical chart of Fig. 18 is enlarged.

Fig. 16 is a statistical chart showing a relationship between an average sensor output voltage value V2 and a specific gravity of a fluid to be discriminated for kerosene, a light oil, a mixed oil A in which kerosene and a light oil are mixed at a ratio of 1:1, a heavy oil, and a mixed oil B in which a heavy oil and a light oil are mixed at a ratio of 1:1 as a fluid to be discriminated. As shown by the statistical chart, kerosene, a light oil, a mixed oil A, and a mixed oil B have a correlation.

Fig. 17 is a statistical chart showing a relationship between an average sensor output voltage value V2 and lubricity (HFRR) of a fluid to be discriminated for kerosene, a light oil, a mixed oil A in which kerosene and a light oil are mixed at a ratio of 1:1, a heavy oil, and a mixed oil B in which a heavy oil and a light oil are mixed at a ratio of 1:1 as a fluid to be discriminated. As shown by the statistical chart, kerosene, a heavy oil, a mixed oil A, and a mixed oil B have a correlation.

Fig. 18 is a statistical chart showing a relationship between an average sensor output voltage value V2 and a coefficient of kinematic viscosity of a fluid to be discriminated for kerosene, a light oil, a mixed oil A in which kerosene and a light oil are mixed at a ratio of 1:1, a heavy oil, and a mixed oil B in which a heavy oil and a light oil are mixed at a ratio of 1:1 as a fluid to be discriminated. As shown by the statistical chart, all types of oils that have measured have a correlation.

Fig. 19 is a statistical chart in which a relationship between an average sensor output voltage value V2 and a coefficient of kinematic viscosity of a fluid to be discriminated for water as a fluid to be discriminated has been added to the graph of Fig. 18. As shown by the statistical chart, an output that is apparently different from that of an oil such as kerosene and a light oil can be obtained in the case in which a fluid to be discriminated is water, and an output that is apparently larger than that of an oil can be obtained in the case in which a coefficient of kinematic viscosity is an index value.

As described above, in the case in which a fluid to be discriminated in the fuel tank 100 is water, an output as an average sensor output voltage value V2 in the case of an oil is in the constant range, thereby enabling a discrimination of a fluid by setting a threshold value.

The embodiment of the present invention shows only the case in which an index value is a coefficient of kinematic viscosity for water as a fluid to be discriminated. However, similarly to the first embodiment, even for other index values, an output as an average sensor output voltage value in the case of an oil is in the constant range, thereby enabling a discrimination of a fluid to be discriminated.

Similarly to the first embodiment of the present invention, a type of a fluid to be discriminated can be discriminated by comparing the fluid discrimination data and the obtained average sensor output voltage value V2.

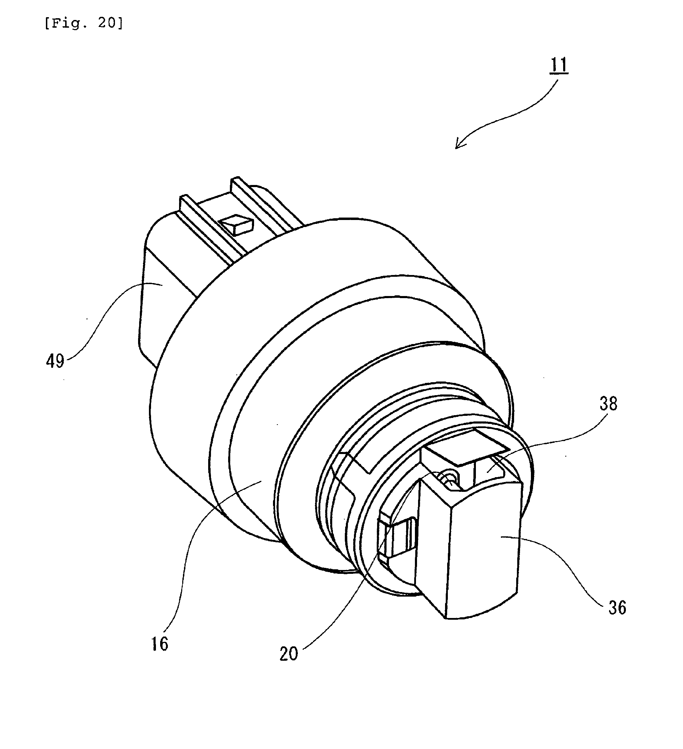

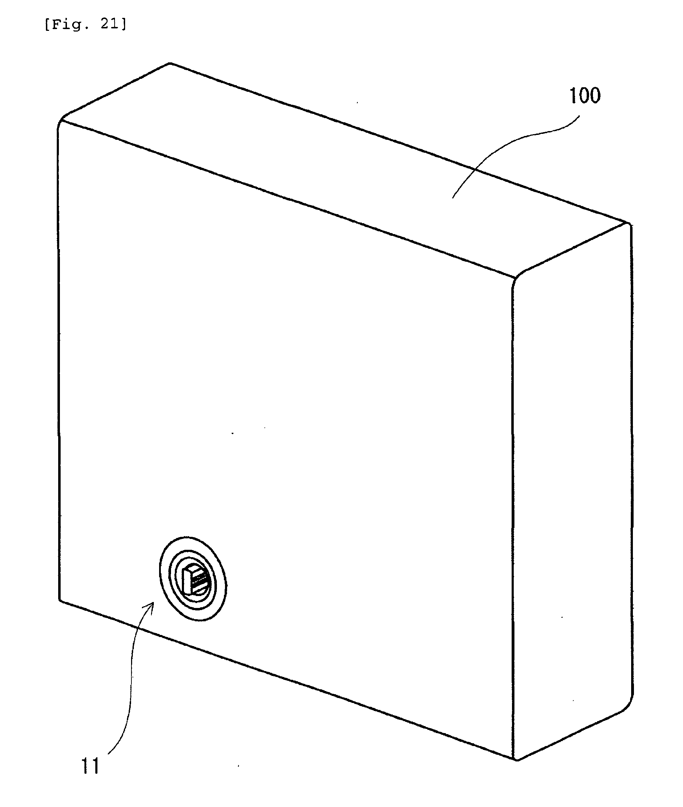

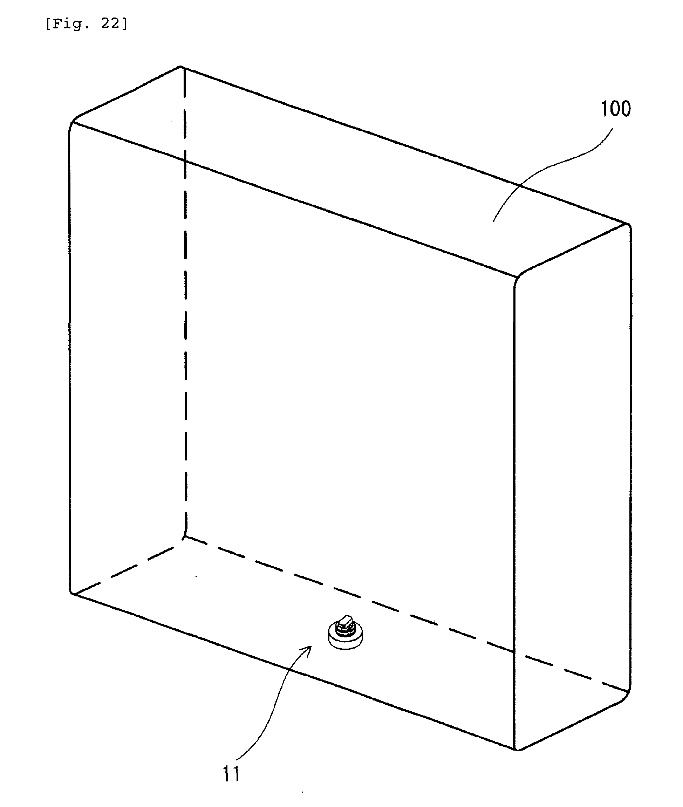

Fig. 20 is a schematic view showing a third embodiment of a fluid discrimination apparatus in accordance with the present invention. Fig. 21 is a schematic view showing an example of a usage state of the fluid discrimination apparatus of Fig. 20. Fig. 22 is a schematic view showing an example of another usage state of the fluid discrimination apparatus of Fig. 20.

In the configuration of a fluid discrimination apparatus 11 in accordance with the present embodiment of the present invention, configuration elements equivalent to those of the fluid discrimination apparatus 10 in accordance with the first embodiment of the present invention are numerically numbered similarly and the detailed descriptions of the equivalent elements are omitted.

In the fluid discrimination apparatus 11 in accordance with the present embodiment of the present invention, a fluid discrimination sensor module 20, a cover member 36, and a communication power connector 49 are disposed in a water proof case 16.

A power cable 40 and a communication cable 42 are connected to the communication power connector 49.

By miniaturizing the fluid discrimination apparatus 11 as shown in Fig. 20, the fluid discrimination apparatus 11 can be attached to the lower side of a fuel tank 100 as shown in Fig. 21 for instance.

As described above, the fluid discrimination apparatus 11 in accordance with the present embodiment of the present invention can be attached to any position of the fuel tank 100. Consequently, a position to which the fluid discrimination apparatus 11 is attached can be changed depending on the type and characteristics of a discriminated fluid that is stored into the fuel tank 100.

As described above, the discrimination accuracy of a fluid to be discriminated can be improved by attaching the fluid discrimination apparatus 11 to the most suitable attachment position depending on the type and characteristics of a fluid to be discriminated.

Moreover, the fluid discrimination apparatus 11 can be attached to the fuel tank 100 in such a manner that the fluid discrimination element 21 and the fluid temperature detecting element 22 of the fluid discrimination sensor module 20 are disposed horizontally to a fluid level as described above.

Moreover, as shown in Fig. 22, the fluid discrimination apparatus 11 in accordance with the present embodiment of the present invention can also be attached to the bottom face of the fuel tank 100 for instance.

In the case in which a discriminated fluid that is stored into the fuel tank 100 is a hydrocarbon series fluid such as a light oil for instance, by attaching the fluid discrimination apparatus 11 to the bottom face of the fuel tank 100 as described above, the fluid discrimination sensor module 20 is dipped into a fluid to be discriminated even when a fuel is consumed unless a fuel in the fuel tank 100 is emptied, whereby the discrimination of a fluid to be discriminated can be carried out at any one time.

Moreover, in the case in which a fluid to be discriminated in the fuel tank 100 is emptied, the sensor output Q is rapidly changed, whereby the existence or nonexistence of a fluid to be discriminated can also be discriminated.

By attaching the fluid discrimination apparatus 11 to the lower side of a fuel tank 100 as shown in Fig. 21, in the case in which the existence or nonexistence of a fluid to be discriminated is discriminated, a user who operates a construction machine can detect that an amount of a fuel in the fuel tank 100 is smaller than the prescribed amount for instance.

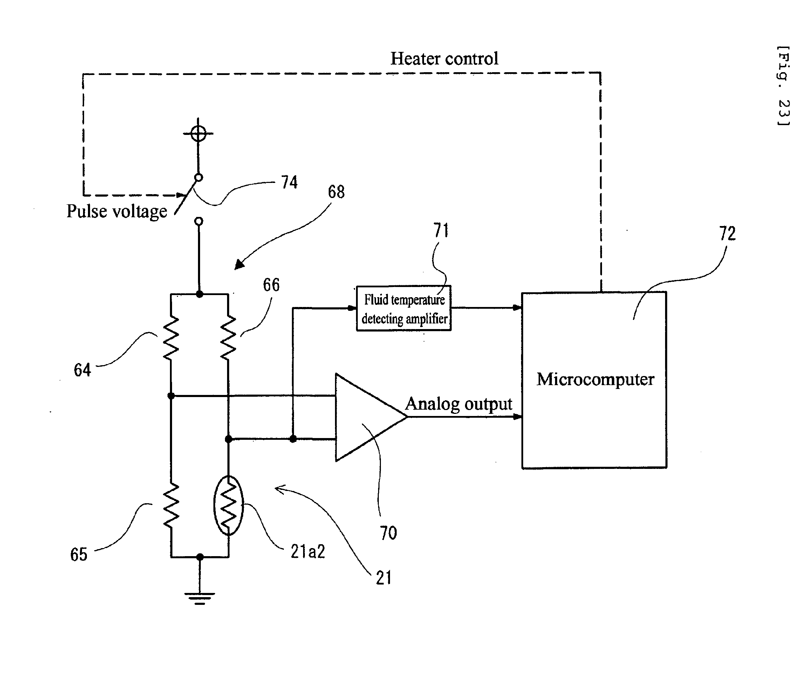

Fig. 23 is another circuit block diagram of an ASIC 52 for a fluid discrimination as a fourth embodiment of a fluid discrimination apparatus in accordance with the present invention.

The configuration of a fluid discrimination apparatus 10 in accordance with the present embodiment of the present invention is basically equivalent to the configuration of a fluid discrimination apparatus 10 in accordance with the first embodiment of the present invention, configuration elements equivalent to those of the fluid discrimination apparatus 10 in accordance with the first embodiment of the present invention are numerically numbered similarly and the detailed descriptions of the equivalent elements are omitted.

As shown in Fig. 23, for the fluid discrimination apparatus 10 in accordance with the present embodiment of the present invention, a power distribution to a bridge circuit (a fluid discrimination circuit) 68 that is configured by the temperature sensing element 21a2 and three resistors 64, 65 and 66 of the fluid discrimination element 21 by controlling the opening and closing of a switch 74 by the power distribution control signal that is output from the microcomputer 72.

In the present embodiment, the temperature sensing element 21a2 of the fluid discrimination element 21 is made of a substance in which an electrical resistance value is changed due to a temperature, such as Pt, Ni, Cr, and W as a metal material, NiCr, FeCr as an alloy material, NiO, FeO, CuO, and Ni₂O₃ as an oxide material, and TaSiO₂ and CrSiO₂ as a cermet.

By the above configuration, by the self heating of the temperature sensing element 21a2, a resistance value of the temperature sensing element 21a2 is changed, a balance of the bridge circuit 68 is broken up, and an output voltage (sensor output) Q is output via the differential amplifier 70 similarly to Fig. 8.

Consequently, similarly to the first embodiment of the present invention, an average sensor output voltage value V1 can be obtained and a fluid discrimination of a fluid to be discriminated can be carried out similarly to the above embodiment of the present invention.

In the case in which the temperature T of a fluid to be discriminated is measured for the present embodiment of the present invention, an electrical output is obtained via a fluid temperature detecting amplifier 71 based on the resistance value of the temperature sensing element 21a2 of the fluid discrimination element 21.

For the present embodiment of the present invention, an output voltage Q is obtained by creating a difference in a temperature change of a resistance value of the temperature sensing element 21a2 and the resistor 65 by using a material different from the resistor 65 as a material of the temperature sensing element 21a2. However, the present invention is not restricted to this configuration. For instance, the output voltage Q can also be obtained by setting a resistance value of the temperature sensing element 21a2 to be larger than a resistance value of the resistor 65 and by setting a calorific value of the temperature sensing element 21a2 to be larger than a calorific value of the resistor 65 to break up a balance of the bridge circuit 68 in accordance with a power distribution to the bridge circuit 68. Moreover, the resistor 65 can also be dipped into the same liquid together with the fluid discrimination element 21 as a fluid temperature detecting element 22 in order to improve the accuracy.

For the present embodiment of the present invention, a pulse voltage is applied to the fluid discrimination element 21 for a prescribed time (10 seconds for instance) by controlling the opening and closing of a switch 74. However, as shown in the second embodiment of the present invention, a voltage can be applied to the fluid discrimination element 21 on a steady basis, and an applied voltage value can be controlled in such a manner that a temperature of a fluid is maintained constant, thereby obtaining a sensor output Q similarly to Fig. 15.

Based on the sensor output Q that has been obtained as described above, a discrimination for a fluid to be discriminated can be carried out similarly to the second embodiment of the present invention.

While the preferred embodiments in accordance with the present invention have been described above, the present invention is not restricted to the embodiments, and various changes, modifications, and functional additions can be thus made without departing from the scope of the present invention. For instance, a pulse voltage value, an application time of a pulse voltage, and a sampling count can be changed as needed.

The present invention is not restricted to the above described embodiments, and various changes, modifications, and functional additions can be thus made without departing from the scope of the present invention. The case in which the fluid discrimination apparatus is attached to a fuel tank of a construction machine or a heavy machine was described in the above embodiments. However, the fluid discrimination apparatus can also be applied to a gasoline tank and an oil tank that stores the lubricating oil for an automobile, and a urea tank for a decomposition of NOx for an automobile.

## Claims

1. A fluid discrimination method for discriminating a fluid to be discriminated, comprising the steps of:
measuring a characteristic measured value as an index value that indicates the characteristics of the fluid to be discriminated; and
carrying out a discrimination of a fluid by comparing the fluid discrimination data that indicates the relationship between a fluid that has been measured in advance and a characteristic value of the fluid with the characteristic measured value.

2. The fluid discrimination method as defined in claim 1, wherein the index value is at least any one of a specific gravity, a coefficient of kinematic viscosity, and a lubricity (HFRR).

3. The fluid discrimination method as defined in claim 1 or 2, wherein a voltage is applied to a fluid discrimination element that includes a temperature sensing element for a prescribed time to heat a fluid to be discriminated, and the characteristic measured value is an electrical output value that is output by a fluid discrimination sensor that outputs an electrical output value corresponded to a temperature of the fluid discrimination element.

4. The fluid discrimination method as defined in claim 1 or 2, wherein a voltage is applied to a fluid discrimination element that includes a temperature sensing element to heat a fluid to be discriminated, and the characteristic measured value is an electrical output value that is based on the voltage and that is output by a fluid discrimination sensor that controls the voltage in such a manner that a temperature of the fluid discrimination element is a constant value.

5. The fluid discrimination method as defined in claim 3 or 4, wherein the fluid discrimination element is provided with an electrical heating element and a temperature sensing element that is disposed close to the electrical heating element.

6. The fluid discrimination method as defined in claim 3 or 4, wherein the temperature sensing element of the fluid discrimination element is a temperature sensing element that is provided with a heating function and a temperature sensing function.

7. The fluid discrimination method as defined in any one of claims 3 to 6, wherein the fluid discrimination sensor is provided with the fluid discrimination element and a fluid temperature detecting element that is disposed separately at a regular interval from the fluid discrimination element.

8. The fluid discrimination method as defined in claim 7, wherein the fluid discrimination element and the fluid temperature detecting element are disposed horizontally to a fluid level.

9. The fluid discrimination method as defined in any one of claims 1 to 8, wherein the discrimination of a fluid to be discriminated is at least one of a fluid type discrimination, a concentration discrimination, the fluid existence or nonexistence discrimination, and a fluid temperature discrimination.

10. The fluid discrimination method as defined in any one of claims 1 to 9, wherein the fluid to be discriminated is a hydrocarbon series fluid.

11. The fluid discrimination method as defined in claim 10, wherein the fluid to be discriminated is at least one of a light oil, a kerosene, and a heavy oil.

12. The fluid discrimination method as defined in claim 11, wherein the fluid to be discriminated is at least one of a light oil, a kerosene, and a heavy oil that are contained in a lubricating oil that is stored into an oil tank.

13. A fluid discrimination apparatus for discriminating a fluid to be discriminated, comprising:
a characteristic measuring part for measuring a characteristic measured value as an index value that indicates the characteristics of the fluid to be discriminated; and
a discrimination control part that carries out a discrimination of a fluid based on the characteristic measured value,
wherein a discrimination of a fluid is carried out by comparing the fluid discrimination data that is stored into the discrimination control part with the characteristic measured value.

14. The fluid discrimination apparatus as defined in claim 13, wherein the index value is at least any one of a specific gravity, a coefficient of kinematic viscosity, and a lubricity (HFRR).

15. The fluid discrimination method as defined in claim 13 or 14, wherein the fluid discrimination data is data that indicates the relationship between a fluid that has been measured in advance and an electrical output value that is measured as an index value that indicates the characteristics of the fluid.

16. The fluid discrimination apparatus as defined in claim 15, wherein:
the characteristic measuring part is provided with a fluid discrimination element that includes a temperature sensing element and a fluid discrimination sensor that outputs an electrical output value corresponded to a temperature of the fluid discrimination element in which a voltage is applied to the fluid discrimination element for a prescribed time to heat a fluid to be discriminated,
the discrimination control part carries out a discrimination of a fluid based on the electrical output value, and
the discrimination control part carries out a discrimination of a fluid by comparing the electrical output value with the fluid discrimination data that is stored into the discrimination control part in advance.

17. The fluid discrimination apparatus as defined in claim 15, wherein:
the characteristic measuring part is provided with a fluid discrimination element that includes a temperature sensing element and a fluid discrimination sensor that controls the voltage in such a manner that a temperature of the fluid discrimination element is a constant value and that outputs an electrical output value that is based on the voltage in which a voltage is applied to the fluid discrimination element to heat a fluid to be discriminated,
the discrimination control part carries out a discrimination of a fluid based on the electrical output value, and
the discrimination control part carries out a discrimination of a fluid by comparing the electrical output value with the fluid discrimination data that is stored into the discrimination control part in advance.

18. The fluid discrimination apparatus as defined in claim 16 or 17, wherein the fluid discrimination element is provided with an electrical heating element and a temperature sensing element that is disposed close to the electrical heating element.

19. The fluid discrimination apparatus as defined in claim 16 or 17, wherein the temperature sensing element of the fluid discrimination element is a temperature sensing element that is provided with a heating function and a temperature sensing function.

20. The fluid discrimination apparatus as defined in any one of claims 16 to 19, wherein the fluid discrimination sensor is provided with the fluid discrimination element and a fluid temperature detecting element that is disposed separately at a regular interval from the fluid discrimination element.

21. The fluid discrimination apparatus as defined in claim 20, wherein the fluid discrimination element and the fluid temperature detecting element are disposed horizontally to a fluid level.

22. The fluid discrimination apparatus as defined in any one of claims 13 to 21, wherein the discrimination of a fluid to be discriminated is at least one of a fluid type discrimination, a concentration discrimination, the fluid existence or nonexistence discrimination, and a fluid temperature discrimination.

23. The fluid discrimination apparatus as defined in any one of claims 13 to 22, wherein the fluid to be discriminated is a hydrocarbon series fluid.

24. The fluid discrimination apparatus as defined in claim 23, wherein the fluid to be discriminated is at least one of a light oil, a kerosene, and a heavy oil.

25. The fluid discrimination apparatus as defined in claim 24, wherein the fluid to be discriminated is at least one of a light oil, a kerosene, and a heavy oil that are contained in a lubricating oil that is stored into an oil tank.
